Europäisches Patentamt

European Patent Office

Office européen des brevets

(11) Numéro de publication: **0 229 542**
**B1**

(12) **FASCICULE DE BREVET EUROPEEN**

(45) Date de publication du fascicule du brevet:
19.09.90

(21) Numéro de dépôt: **86402542.4**

(22) Date de dépôt: **17.11.86**

(51) Int. Cl.⁵: **A61F 13/15**

(54) Procédé pour former une dépression à la surface d'une structure absorbante.

(30) Priorité: **20.11.85 FR 8517128**

(43) Date de publication de la demande:
**22.07.87 Bulletin 87/30**

(45) Mention de la délivrance du brevet:
**19.09.90 Bulletin 90/38**

(84) Etats contractants désignés:
**BE CH DE ES FR GB GR IT LI LU NL**

(56) Documents cités:
**EP-A- 0 067 916**
**EP-A- 0 173 068**
**FR-A- 2 350 830**
**FR-A- 2 354 753**
**GB-A- 2 023 067**
**US-A- 3 881 490**

(73) Titulaire: **KAYSERSBERG SA, Route de Lapoutroie,
F-68240 Kaysersberg(FR)**

(72) Inventeur: **Pierre, Michel, 2, Passage de l'Hôtel de Ville,
F-68100 Mulhouse(FR)**

(74) Mandataire: **David, Daniel, KAYSERSBERG 54, avenue
Hoche, F-75008 Paris(FR)**

ACTORUM AG

## Description

L'invention se rapporte à une structure absorbante appliquée à l'hygiène corporelle, telle que serviette périodique, couche pour bébé, change complet ou tout autre article d'hygiène destiné à absorber les liquides corporels, comportant sur sa face supérieure au moins une rainure, un canal ou toute autre forme de dépression. L'invention vise en particulier un procédé pour former cette dépression.

Les structures absorbantes sont composées, de façon connue, d'au moins un noyau absorbant recouvert d'un revêtement perméable côté corps, et d'une feuille imperméable sur la face opposée. Le revêtement peut se prolonger sur la face opposée et recouvrir aussi la feuille imperméable. Le revêtement est communément en non-tissé, et la feuille imperméable réalisée dans un film de polyéthylène par exemple. Le noyau absorbant est généralement constitué de fibres cellulosiques telles que des fibres papetières, obtenues par défibrage à sec d'une feuille de pâte à papier, appelées fluff. Il peut être réalisé aussi, à partir de sphaigne ou toute autre matière absorbante. Il peut contenir des particules d'hydrorétenteurs qui sont des macromolécules hydrophiles insolubles, capables de gélifier les fluides aqueux et physiologiques. Il peut contenir également des matières ayant pour fonction de renforcer l'article ou de favoriser la diffusion des liquides : non-tissé, colle, papier paraffiné, fibres synthétiques, etc...

Afin d'améliorer les propriétés d'absorption des articles d'hygiène à usage unique, il est connu de ménager un ou plusieurs canaux sur la face supérieure du noyau absorbant.

Ainsi, le brevet FR 1590071 décrit une serviette périodique dont le noyau comporte sur sa face supérieure une dépression centrale longitudinale de faible largeur par rapport à celle de la serviette. La fonction essentielle de ce canal est de lui conférer, en usage, une structure latérale à concavité dirigée vers le haut offrant les avantages d'un positionnement correct, d'une limitation des risques de maculage et d'un confort accru pour l'utilisatrice.

La dépression centrale est réalisée par compression de la matière au moyen d'un outil de forme appropriée. La masse de fibres située sous le canal ou dépression est alors plus dense ; les liquides sont absorbés par effet de mèche en cheminant plus rapidement le long du réseau de capillaires plus fins dans cette zone, que latéralement par les capillaires des zones non comprimées. Il en résulte une meilleure distribution des fluides dans la masse absorbante et une utilisation plus complète de la capacité d'absorption. Selon ce brevet, la masse absorbante est constituée de fibres papetières, de linters de coton, liées entre elles suivant un procédé à base de mousse, de manière à obtenir une structure fibreuse expansée.

La demande de brevet EP 124365 décrit une structure absorbante dans laquelle sont ménagés deux réservoirs reliés par un canal. Ces cavités sont obtenues par compression, au moyen d'un poinçon, de la masse de fibres compactées formant le noyau. On prévoit la création de liaisons interfibres dans la couche formant la base des cavités, pour éviter que les fibres ne tendent à réoccuper leur volume initial sous l'effet des forces élastiques qui leur sont propres. A cet effet, on humidifie préalablement le noyau avant l'opération de compression.

La demande de brevet EP 106473 décrit un protège-slip présentant en surface, une pluralité de dépressions imprimées profondément dans la matière absorbante. La stabilité dimensionnelle de ces motifs est rendue possible par la composition de la matière absorbante qui comprend un mélange de longues fibres de rayonne et de courtes fibres papetières, la cohésion étant assurée par l'incorporation d'un liant pouvant représenter 30% de la masse fibreuse.

Lorsque le noyau absorbant est en mousse de cellulose, c'est-à-dire constitué de fibres papetières obtenues par défibrage à sec de pâte à papier, essentiellement non liée, ni compactée, sa structure est duveteuse, sans consistance, n'offrant aucune cohésion. Il est difficile d'assurer une stabilité dimensionnelle satisfaisante à toute dépression réalisée dans ce duvet. Après formation de la rainure, le revêtement se désolidarise du noyau à la moindre sollicitation.

Une solution a consisté alors à déposer de la colle entre le revêtement et le noyau, avant d'appliquer l'outil de marquage pour obtenir la dépression désirée. Une telle solution est décrite dans le brevet US 2 788 003 où l'on utilise de la colle thermofusible et où le calandrage est effectué à chaud. Dans la demande EP 67 916 également, le liant est un constituant thermoplastique du revêtement qui au moment du pressage à chaud assure la liaison avec le noyau absorbant. Toutefois, sauf à en ajouter une quantité prohibitive contrariant la fonction d'absorption du noyau, on constate que la colle ne migre pas et ne parvient pas à former une liaison au niveau de la rainure, entre le revêtement perméable et la face arrière, surtout lorsque le revêtement est constitué de matières plastiques hydrophobes qui se lient mal aux fibres cellulosiques. Pour pallier cette difficulté on a imaginé de placer une feuille d'ouate de cellulose sous le revêtement et de la coller à la fois à celui-ci et au noyau absorbant. Cependant, même dans ce cas la liaison n'évite pas la déformation de la rainure, et la feuille d'ouate de cellulose ne favorise pas la pénétration des liquides dans le noyau.

L'invention se propose de réaliser au moins une dépression sur la face supérieure d'une structure absorbante constituée notamment de fibres hydrophiles non compactées et non liées, présentant une stabilité dimensionnelle satisfaisante.

Par rapport à un procédé pour former au moins une dépression sur la face supérieure d'un article d'hygiène, tel qu'une serviette périodique, du type comprenant un noyau absorbant composé essentiellement de fibres hydrophiles non liées, présentant une face supérieure et une face inférieure, un revêtement supérieur perméable aux liquides recouvrant la face supérieure et adhérant au noyau absorbant au moyen d'un liant, une feuille imperméable aux liquides recouvrant la face inférieure, le revêtement et la feuille étant disposés de manière à enfermer le noyau absorbant, le procédé de l'invention est caractérisé en ce qu'il consiste, en cours de fabrication avant que le revêtement et la feuille imperméable ne soient associés au noyau absorbant, à déposer une substance adhésive sur le revêtement pour former ledit liant et une substance adhésive sur la feuille imperméable sur la partie de leur surface, tournée vers le noyau, située dans une zone déterminée, puis après confection de l'article, à appliquer un outil de formation de la dépression sur ladite zone avec une pression suffisante pour que les deux surfaces adhésivées se lient l'une à l'autre.

Le dépôt de la substance adhésive peut être continu ou bien seulement par points ou par lignes qui se font face afin qu'ils constituent les deux extrémités de la liaison entre le revêtement et la feuille imperméable.

Il est possible de réaliser une préforme de la dépression dans le noyau absorbant, par exemple par enlèvement de matière avant le recouvrement du noyau par le revêtement. Le défaut de matière absorbante peut alors être réalisé lors de la formation de la nappe sur un tambour alvéolé, par un peignage non uniforme de la nappe.

Grâce à ce procédé on conserve à l'article ses propriétés dues à l'utilisation de fibres non liées, qui sont douceur, grande capacité d'absorption, confort d'utilisation, tout en obtenant les avantages résultant de la formation d'une ou plusieurs dépressions sur la face supérieure de l'article, qui sont pour une serviette périodique, une meilleure diffusion des liquides à travers toute la masse absorbante et une déformation contrôlée sous l'action de forces de pression latérales.

L'invention sera mieux comprise à la lecture de la description d'un mode de réalisation non limitatif de l'invention en référence aux dessins où:

La figure 1 représente une serviette périodique obtenue selon le procédé de l'invention, vue de dessus.

La figure 2 est une vue en coupe selon II II de la figure 1.

La figure 3 représente de façon schématique et partielle un exemple d'installation permettant la réalisation d'une serviette avec rainure longitudinale centrale.

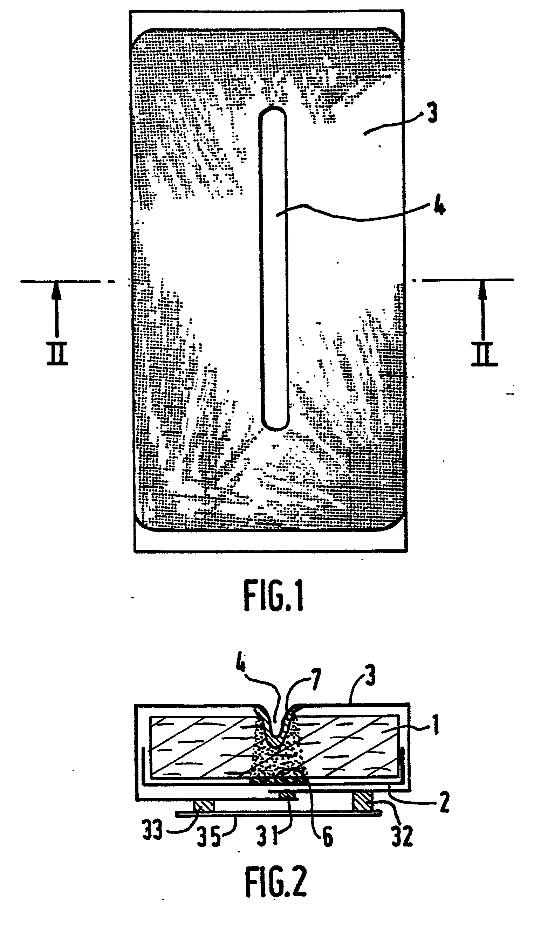

Sur la figure 1 et 2 est représentée une serviette périodique de forme sensiblement rectangulaire, comprenant un noyau absorbant 1 constitué essentiellement de fibres papetières, avec lesquelles sont mélangés, éventuellement, en faible quantité, des superabsorbants. Une feuille de polyéthylène 2, imperméable, est disposée sur la face inférieure par rapport au dessin. Cette feuille s'étend sur toute cette face et déborde légèrement le long des deux faces latérales du noyau. L'ensemble est enveloppé dans un revêtement qui couvre la face supérieure et qui, dans cet exemple, est suffisamment large pour envelopper également la feuille imperméable. Les bords longitudinaux du revêtement sont solidarisés l'un à l'autre au moyen d'un trait de colle 31. De même, les bords transversaux sont scellés entre eux à chaque extrémité. De cette façon, le noyau est enfermé entièrement dans le revêtement 3. Des traits de colle 32 et 33 peuvent être prévus à l'extérieur de la face inférieure pour permettre le maintien de la serviette au sous-vêtement. Avant usage, ces traits de colle sont protégés par un papier siliconé 35. Le revêtement est un non-tissé perméable aux liquides corporels, de préférence hydrophobe pour éviter la formation de taches.

Une rainure 4 est ménagée sur la face supérieure de la serviette. Cette rainure est disposée centralement, dans le sens longitudinal. Sa largeur est suffisante pour lui permettre de remplir la fonction qui lui est dévolue, à savoir canalisation des fluides et déformation dirigée de la serviette. Par exemple elle peut être de l'ordre de 1/10 de la largeur de la serviette.

La longueur de la rainure est inférieure à celle de la serviette afin d'éviter les fuites de liquide par les extrémités. Enfin sa profondeur est égale à au moins 20 % de l'épaisseur de la serviette de préférence de l'ordre de 50 %.

Conformément à l'invention, afin de garantir la tenue de cette rainure, on prévoit de lier le revêtement à la feuille imperméable à travers la masse absorbante. A cette fin on dépose, au cours de la fabrication de la serviette, une substance adhésive 6 sur la feuille 2 et une substance adhésive 7 sur le revêtement 3 à un emplacement correspondant sensiblement à celui de la rainure. L'adhésif est un produit couramment utilisé pour ce type d'article par exemple il peut s'agir d'un polyvinyl-alcool, ou d'une colle thermofusible du type dit hot melt. Dans ce dernier cas il faut s'assurer que la colle soit suffisamment fluide au moment du dépôt et du marquage.

La quantité moyenne de colle déposée sur chaque face (en extrait sec) est de l'ordre de 2 à 70 g/m² et de préférence de 5 à 20 g/m², c'est-à-dire de l'ordre de 10g/m² par multitraits étroits et de 5 g/m² en enduction plus large. L'application n'est pas nécessairement uniforme.

Après application de l'adhésif, on dispose le revêtement et la feuille sur le noyau et on confectionne, de façon connue, la serviette.

Enfin on applique l'outil permettant le marquage de la rainure avec une pression suffisante pour que les substances adhésives 6 et 7 migrent l'une vers l'autre à travers les fibres comprimées par l'outil et forment une liaison entre le revêtement et la feuille, après séchage de la colle. Dans une variante, la rainure peut être déjà préformée dans le noyau absorbant dès la formation de ce dernier, par enlèvement de matière. L'outil de marquage est appliqué ensuite sur le revêtement au niveau de cette préforme.

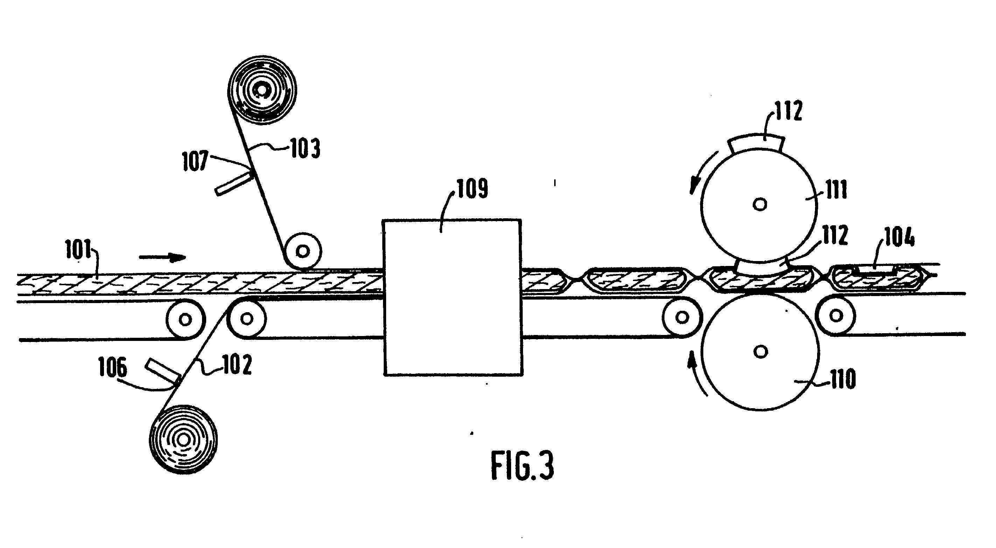

Sur la figure 3 est représentée schématiquement et partiellement une installation pour la fabrication en chaîne de serviettes périodiques. La matière absorbante 101 est distribuée sur un convoyeur à bande par une trémie située en amont de la chaîne, non représentée. Le ruban continu ainsi formé est transféré sur un film 102 en polyéthylène, lui-même en déplacement sur un convoyeur à bande. Ce film constitue la feuille imperméable de la serviette. De la colle 106 est déposée sur le film selon un trait continu disposé centralement, en un point situé en amont de l'endroit où le film rejoint le ruban de matière absorbante. Sur la face supérieure du ruban est déposé de la même façon un film de non-tissé hydrophobe 103 devant constituer le revêtement. Préalablement à sa jonction avec le ruban 101 de fibres, un trait de colle 107 a été déposé centralement, sur la face du non-tissé devant venir au contact du ruban. Au poste 109 situé en aval, le ruban est enveloppé dans le non-tissé, les bords longitudinaux de ce dernier sont collés l'un à l'autre puis les serviettes sont formés et les extrémités de l'enveloppe scellées. Les serviettes, formant un chapelet, passent ensuite entre deux cylindres à axes parallèles 110 et 111 disposés transversalement au sens machine. Le premier cylindre 110 est lisse, et le second cylindre 111 comporte un ergot 112 conformé de façon à assurer le marquage de la dépression 104 sur la serviette. Dans l'exemple représenté, l'ergot a la forme d'une lame dont l'épaisseur correspondant à celle de la rainure sur les serviettes, la lame fait saillie à la surface du cylindre sur laquelle elle s'étend en arc de cercle. Deux ergots sont représentés, mais il est possible d'en prévoir un nombre différent, ils sont disposés sur un même plan de cercle. Le nombre de serviettes marquées par tour de cylindre est égal au nombre des ergots. L'espacement entre l'arête de l'ergot 112 et le cylindre 110 est choisi afin de garantir une compression suffisante du matelas, et permettre la formation de la liaison entre le revêtement 103 et la feuille 102 par l'intermédiaire des dépôts de colle 106 et 107. Après cette étape, les serviettes sont séparées les unes des autres et conditionnés pour la vente.

Le mode de réalisation représenté concerne une seule rainure longitudinale centrale, mais l'invention s'applique bien évidemment à toute forme de dépression ménagée sur la face supérieure de l'article.

De même l'invention n'est pas seulement applicable aux serviettes périodiques mais aussi aux couches pour bébé ou aux changes complets, etc...

L'enveloppe de l'article est constituée dans cet exemple par le revêtement permeable, elle peut cependant comprendre la feuille imperméable ; auquel cas les deux feuilles, permeable et imperméable, sont soudées l'une à l'autre le long des bords longitudinaux.

Si le noyau absorbant est composé de deux nappes de mousse de cellulose superposées, la jonction adhésive à l'endroit de la rainure peut être encore consolidée par l'introduction d'un non-tissé ou d'un film permeable collé entre les deux nappes comme il est décrit dans la demande de brevet français 8503016.

La stabilité dimensionnelle est apportée par la jonction par adhésifs du revêtement à la face arrière. L'adhésif appliqué sur une face peut être différent de celui appliqué sur l'autre : par exemple, l'adhésif côté revêtement peut être hydrophile (colle polyacrylate, latex...) et celui sur la face arrière hydrophobe. L'adhésif servant à la jonction peut être constitué de deux composants A et B déposés séparément, l'un A sur la face arrière, l'autre B côté revêtement. La réaction de A avec B formera le collage et la liaison. Par exemple on pourra déposer sur la face arrière une colle (polyuréthane) activable par l'eau, par exemple celle connue sous la marque DURELAST-4055. L'eau sera alors apportée par l'adhésif déposé côté revêtement, par exemple un latex.

Des essais comparatifs ont été réalisés pour vérifier les résultats de l'invention. A cette fin, on a préparé selon le procédé décrit ci-dessus une première serviette périodique dont le revêtement est un non-tissé hydrophobe en fibres de polypropylène, vendu sous la marque HOLNEST. Le noyau est en mousse de cellulose, et la face opposée au revêtement est une feuille de polyéthylène. On a utilisé une colle hot-melt de la Société BERICOL référencée BELLIX 74101. Le dépôt sur chaque face est de 10 g/m² environ. Le non-tissé est encollé de 16 traits de 1 mm de large espacés de 3 mm et le polyéthylène de 3 traits de 3 mm de large.

On a préparé de la même façon une deuxième serviette périodique, sans déposer cette fois-ci de colle sur le polyéthylène. Afin d'obtenir une certaine tenue de la rainure il a été nécessaire d'augmenter de 50 % le débit de colle sur le revêtement supérieur.

On a mesuré le pourcentage de gouttes de 0,20 ml de sang de formule : 85 % de sang de bovin, 15 % d'eau salée à 9 g/l de chlorure de sodium, additionné de traces d'héparinate de lithium comme anticoagulant qui, déposées sur le revêtement pénètrent d'elles-mêmes dans des serviettes périodiques en moins de trente secondes.

Dans le premier cas, le taux de pénétration du sang est de 100 % sur les bords et de 80 % dans la rainure. Le revêtement adhère solidement au noyau.

Dans le deuxième cas le taux de pénétration des gouttes de sang est de 90 % sur les bords et de 10 % seulement dans la rainure. Cette mauvaise pénétration dans la rainure est consécutive à l'augmentation de la quantité de colle hot-melt de 50 % environ. Malgré cela, le non-tissé se décclle facilement lorsque la serviette est manipulée.

On constate que l'amélioration dans le premier cas résulte de la répartition de la colle sur les deux faces permettant de réaliser une meilleure jonction adhésive et de conserver une absorption rapide dans la rainure en évitant le colmatage de revêtement avec un apport trop important d'adhésif.

**Revendications**

1. Procédé pour former au moins une dépression (4) sur la face supérieure d'un article d'hygiène, tel qu'une serviette périodique, du type comprenant un noyau absorbant (1) composé essentiellement de fibres hydrophiles non liées, présentant une face supérieure et une face inférieure, un revêtement supérieur (3) permeable aux liquides recouvrant la face supérieure et adhérant au noyau absorbant au moyen d'un liant, une feuille imperméable (2) aux liquides recouvrant la face inférieure, le revêtement et la feuille étant disposés de manière à enfermer le noyau absorbant, caractérisé en ce qu'il consiste, en cours de fabrication avant que le revêtement et la feuille imperméable ne soient associés au noyau absorbant, à déposer une substance adhésive (7) sur le revêtement pour former ledit liant et une substance adhésive (6) sur la feuille imperméable sur la partie de leur surface, tournée vers le noyau, située dans une zone déterminée, puis après confection de l'article, à appliquer un outil de formation de la dépression sur ladite zone avec une pression suffisante pour que les deux surfaces adhésivées se lient l'une à l'autre.

2. Procédé selon la revendication 1, caractérisé en ce que les substances adhésives (6, 7) sont déposées en traits parallèles continus.

3. Procédé selon la revendication 2, caractérisé en ce que les substances adhésives (6, 7) sont déposées par points.

4. Procédé selon l'une des revendications précédentes, caractérisé en ce que la quantité de substance adhésive déposée sur chaque face (revêtement 3 et feuille imperméable 2) est comprise entre 5 et 20 g/m² (en extrait sec).

5. Procédé selon l'une des revendications précédentes, caractérisé en ce que la substance adhésive déposée sur le revêtement est différente de celle qui est déposée sur la feuille imperméable.

6. Procédé selon l'une des revendications précédentes, caractérisé en ce qu'une préforme de la dépression est réalisée dans le noyau absorbant par enlèvement de matière, avant que le revêtement ne soit associé audit noyau.

7. Procédé selon l'une des revendications précédentes, caractérisé en ce que l'on fait passer l'article entre deux cylindres (110, 111) dont l'un est pourvu d'un ergot (112) conformé en fonction de la dépression voulue.

8. Procédé pour former une dépression sur la face supérieur d'une serviette périodique selon la revendication 7, caractérisé en ce que l'ergot est en forme de lame.

## Claims

1. A process for forming at least one depression (4) on the top side of a hygiene article such as a sanitary towel of the type having an absorbent core (1) consisting essentially of non-bonded hydrophilic fibres, having an upper side and a lower side, an upper covering (3) which is permeable to liquids covering the upper side and adhering to the absorbend core by means of a binder, a sheet (2) which is impermeable to liquids covering the lower side, the covering and the sheet being positioned in such a way as to enclose the absorbent core, characterised in that it consists of depositing an adhesive substance (7) onto the covering to form the said binder and an adhesive substance (6) onto the impermeable sheet in a particular area of the parts of their surfaces facing the core during the course of manufacture, before the covering and the impermeable sheet are associated together with the absorbent core, followed by the application, after assembly of the article, of a depression-forming tool to the said zone with sufficient pressure for the two adhesive-coated surfaces to bond together.

2. A process according to claim 1, characterised in that the adhesive substances (6, 7) are deposited as continuous parallel lines.

3. A process according to claim 2, characterised in that the adhesive substances (6, 7) are deposited as spots.

4. A process according to one of the foregoing claims, characterised in that the quantity of adhesive substance deposited on each side (covering 3 and impermeable sheet 2) lies between 5 and 20 g/m² of dry extract.

5. A process according to one of the foregoing claims, characterised in that the adhesive substance deposited on the covering is different from that deposited on the impermeable sheet.

6. A process according to one of the foregoing claims, characterised in that a preformed depression is made in the absorbent core by the removal of material before the covering is associated with the said core.

7. A process according to one of the foregoing claims, characterised in that the article is passed between two rollers (110, 111), one of which is provided with a projection (112) shaped according to the desired depression.

8. A process for forming a depression on the upper side of a sanitary towel according to claim 7, characterised in that the projection is in the shape of a blade.

## Patentansprüche

1. Verfahren zum Formen einer Vertiefung (4) auf der Oberseite eines Hygieneartikels, wie eine Damenbinde, vom Typ, der einen absorbierenden Kern (1) enthält, der im wesentlichen aus hydrophilen, unverbundenen Fasern zusammengesetzt ist, der eine Oberseite und eine Unterseite aufweist, der eine obere Verkleidung (3) enthält, die für Flüssigkeiten durchlässig ist, die die Oberseite bedeckt und die an dem absorbierenden Kern mittels eines Bindemittels festklebt, der eine für Flüssigkeiten undurchlässige Folie (2) enthält, die die Unterseite bedeckt, wobei die Verkleidung und die Folie so angeordnet sind, daß sie den absorbierenden Kern einschließen, dadurch gekennzeichnet, daß es darin besteht, während der Herstellung bevor die Verkleidung die undurchlässige Folie mit dem absorbierenden Kern verbunden sind, eine klebende Substanz (7) auf der Verkleidung abzulagern, um das Bindemittel zu bilden und eine klebende Substanz (6) auf der undurchlässigen Folie auf dem Teil ihrer Oberfläche abzulagern, der dem Kern zugewandt ist, der in einem bestimmten Bereich gelegen ist, dann nach der Konfektion des Artikels ein Werkzeug zum Formen der Vertiefung auf der Zone anzusetzen, mit einem ausreichenden Druck, damit die beiden klebenden Oberflächen sich miteinander verbinden.

2. Verfahren gemäß Anspruch 1, dadurch gekennzeichnet, daß die klebenden Substanzen (6, 7) in ununterbrochenen parallelen Linien abgelagert sind.

3. Verfahren gemäß Anspruch 2, dadurch gekennzeichnet, daß die klebenden Substanzen (6, 7) durch Punkte abgelagert sind.

4. Verfahren gemäß einem der vorstehenden Ansprüche, dadurch gekennzeichnet, daß die Menge der abgelagerten klebenden Substanz auf jeder Seite (Verkleidung 3 und undurchlässige Folie 2) zwischen 5 und 20 g/m² (im Trockenextrakt) enthalten ist.

5. Verfahren gemäß einem der vorstehenden Ansprüche, dadurch gekennzeichnet, daß die klebende Substanz, die auf der Verkleidung abgelagert ist, verschieden von der ist, die auf der undurchlässigen Folie abgelagert ist.

6. Verfahren gemäß einem der vorstehenden Ansprüche, dadurch gekennzeichnet, daß eine Vorform der Vertiefung in dem absorbierenden Kern durch Abheben vom Material ausgeführt ist, bevor die Verkleidung mit dem Kern verbunden ist.

7. Verfahren gemäß einem der vorstehenden Ansprüche, dadurch gekennzeichnet, daß man den Artikel zwischen zwei Zylindern (110, 111) passieren läßt, wovon einer mit einem Vorsprung (112) versehen ist, der in der Funktion der beabsichtigten Vertiefung entspricht.

8. Verfahren zum Formen einer Vertiefung auf der Oberseite einer Binde gemäß Anspruch 7, dadurch gekennzeichnet, daß der Vorsprung Klingenform hat.

FIG.1

FIG.2

FIG.3

EP 0 229 542 B1